# EUROPÄISCHE PATENTSCHRIFT

(11) **EP 0 574 410 B1**
(45) Veröffentlichungstag und Bekanntmachung des Hinweises auf die Patenterteilung: **22.05.1996**
(21) Anmeldenummer: 92903227.4
(22) Anmeldetag: 20.01.1992
(51) Int. Cl.: A61J 1/05

(54) **BLUTBEUTELANORDNUNG**
BLOOD-BAG SYSTEM
SYTEME DE SACS DE SANG

(30) Priorität: 07.03.1991 DE 9102709 U
(43) Veröffentlichungstag der Anmeldung: 22.12.1993
(73) Patentinhaber: BLUTSPENDEDIENST DER LANDESVERBÄNDE DES DEUTSCHEN ROTEN KREUZES NIEDERSACHSEN, OLDENBURG UND BREMEN G.G.m.b.H., D-31830 Springe (DE)
(72) Erfinder: MOHR, Harald, 30177 Hannover (DE)
(74) Vertreter: Schupfner, Gerhard D.
(86) Internationale Anmeldenummer: DE9200031
(87) Internationale Veröffentlichungsnummer: WO9215274

(56) Entgegenhaltungen:
- EP-A- 0 196 515
- EP-A- 0 382 018
- WO-A-87/06119
- WO-A-91/03933

## Beschreibung

Die Erfindung bezieht sich auf eine Blutbeutelanordnung für Spenderblut und/oder Blutprodukte mit einem Beutel,zumindest einem an diesen angeschlossenen Schlauch und einer Einrichtung zur Inaktivierung der Viren, wobei die Anordnung aus zumindest teilweise lichtdurchlässigem Kunststoffmaterial besteht.

Aus der WO-A-87 06 119 und der EP-A-0 382 018 sind Blutbeutelanordnungen bekannt, mit deren Hilfe Spenderblut gewonnen und weiterverarbeitet wird. Die Blutbeutelanordnungen bestehen aus einem Entnahme- und mehreren weiteren angeschlossenen Beutel(n), die durch Schläuche miteinander verbunden sind. Die Beutel dienen zum einen der Aufnahme von Blut oder Blutfraktionen.

Problematisch ist beim Arbeiten mit Blutbeuteln insbesondere, daß das in die sterilen Blutbeutel eingeführte Präparat Viren enthalten kann, die inaktiviert werden müssen.

Die EP-A-0382 018 und die WO-A-87 06 119 beschreiben an solchen Blutbeutelanordnungen über flexible Schläuche angeschlossene flexible Behälter, in denen eine neutralisierende Substanz als Flüssigkeit oder Pulver enthalten ist. Die WO-A-87 06 119 erwähnt auch das Einbringen der neutralisierenden Substanzen unmittelbar in die Beutel. In den zusätzlichen Behältern sind die Substanzen zunächst eingeschlossen. Durch das Öffnen oder Brechen von Ventilen können sie dann mit dem Inneren der Blutbeutel in Kontakt kommen. Befinden sich die Substanzen gleich in den Beuteln, dann werden sie unmittelbar aktiv.

Die EP-A-0 196 515 und die WO-A-91 03 933 beschreiben Verfahren zum Inaktivieren von Viren in Blut und Blutprodukten, bei denen die zu behandelnden Lösungen mit photoaktiven Substanzen versetzt und anschließend bestrahlt werden.

Die Beutel und die Schläuche bestehen aus flexiblem Kunststoff. Die Kunststoffe der Beuteln und Schläuchen sind zumindest teilweise lichtdurchlässig.In ihnen wird das Spenderblut durch Zentrifugation aufgetrennt, z. B. in Plasma, Thrombozyten und Erythrozyten.

Es ist Aufgabe der Erfindung, eine Blutbeutelanordnung zu schaffen, in der das Blut bzw. Blutbestandteile einzelner Spender virussicher gemacht werden kann/können bzw. virussicher untergebracht ist/sind, wobei die Mittel, die das Blut oder die Blutbestandteile virussicher machen, bis zu ihrer Inbenutzungnahme in der benuzungsbereiten, als selbstständige Einheit einzeln handhabbaren Anordnung in abgeschlossenen Bereichen eingeschlossen sind, wobei die Mittel in für die Blutbehandlung notwendigen Teilen der Anordnung bevorratet sind.

Die gestellte Aufgabe ist bei einer Blutbeutelanordnung für Spenderblut und/oder Blutprodukte mit einem Beutel und zumindest einem an diesen angeschlossenen Schlauch, wobei die Anordnung aus zumindest teilweise lichtdurchlässigem Kunststoffmaterial besteht, erfindungsgemäß dadurch gelöst, daß in einem abgeschlossenen Bereich des Schlauches der benutzungsbereiten Anordnung, die als selbstständige Einheit einzeln handhabbar ist, photoaktive Substanzen (31) einschließbar sind.

Die gestellte Aufgabe ist ebenso bei einer Blutbeutelanordnung für Spenderblut und/oder Blutprodukte mit mehreren Beuteln und an diesen angeschlossene Schläuchen, die aus zumindest teilweise lichtdurchlässigem Kunststoffmaterial bestehen, wobei die Schläuche die Beutel miteinander verbinden und/oder an anderweitige Schläuche angeschlossen sind, erfindungsgemäß dadurch gelöst, daß in abgeschlossenen Bereichen der Schläuche der benutzungsbereiten Anordnung, die als selbständige Einheit einzeln handhabbar ist, photoaktive Substanzen einschließbar sind.

Werden während der Herstellung der Blutbeutelanordnungen in deren Schläuchen photoaktive Substanzen angeordnet, und werden diese Substanzen in der Blutbeutelanordnung mit sterilisiert, dann kann nach der Inbenutzungnahme in den Blutbeutelanordnungen mit diesen Zusätzen eine Behandlung, insbesondere eine Virusinaktivierung des Spenderblutes oder Blutplasmas vorgenommen werden, indem die Blutbeutelanordnungen nach dem Vermischen des Spenderblutes mit den photoaktiven Substanzen belichtet werden. Die photoaktiven Substanzen haben die Eigenschaft, vom Licht angeregt zu werden und Energie abzugeben, was dazu führt, daß eventuell in dem Präparat vorhandene Viren inaktiviert werden.

Die Aufgabe ist weiterhin bei einer Blutbeutelanordnung für Spenderblut und/oder Blutprodukte mit zumindest einem Beutel, zumindest einem angeschlossenen Schlauch, über den die Beutel weiterverbindbar sind, und einer Einrichtung zur Inaktivierung von Viren, wobei die Anordnung zumindest teilweise aus lichtdurchlässigem Kunststoffmaterial besteht, erfindungsgemäß dadurch gelöst, daß eine selbstständig und einzeln zu handhabende Blutbeutelanordnung, bestehend aus einem Beutel und einem in abgeschlossenen Bereichen photoaktive Substanzen einschließenden Schlauch, mit einer anderen selbständig und einzeln zu handhabenden Blutbeutelanordnung, die aus einem Beutel und einem Schlauch oder aus mehreren Beuteln und die Beutel verbindenden Schläuchen besteht, kombinierbar ist durch ein Miteinanderverbinden dieser Blutbeutelanordnungen.

Eine der beiden Blutbeutelanordnungen kann also frei von photoaktiven Substanzen sein. Trotz vollwirksamer Virusinaktivierung kann damit eine der kombinierten Anordnungen wirtschaftlicher bereitgestellt werden.

Das Kombinieren kann in der Weise durchgeführt werden, daß ein Schlauch der einen Blutbeutelanordnung an einen Schlauch der anderen Blutbeutelanordnung mit Hilfe eines Schweißapparates unter sterilen Bedingungen angekoppelt wird.

Nach einer weiteren Ausgestaltung der zweiten Ausführungsform der Erfindung ist vorgesehen, daß bei einer Mehrbeutelanordnung Entnahmebeutel und weitere Beutel über Substanzen einschließende Schläuche miteinander verbunden sind. So kann die Behandlung des Spenderblutes in einem von vornherein in sich geschlossenen System erfolgen.

Die Substanzen können in den Schläuchen mittels Brechventilen eingeschlossen sein. Durch das Einschließen in den Schläuchen wird sichergestellt, daß die Substanzen überall bereit sind, mit dem Blut oder den Blutfraktionen in Berührung zu kommen. Durch das Öffnen der Schlauchverbindungen infolge eines Brechens der Brechventile lassen sich die eingeschlossenen Substanzen gezielt mit dem Blut oder den Blutfraktionen in Verbindung bringen.

Nach einer bevorzugten Ausgestaltung der Erfindung sind die photoaktiven Substanzen im flüssigen bzw. gelösten Zustand vorgesehen. Der flüssige Aggregatzustand erlaubt ein besonders rasches Vermischen der photoaktiven Substanzen mit dem Blut oder den Blutfraktionen.

Nach einer weiteren Ausgetaltung der Erfindung ist vorgesehen, daß als photoaktive Substanzen Phenothiazinfarbstoffe vorgesehen sind, und zwar vorzugsweise Methylenblau oder Toluidinblau.

Die Erfindung wird anhand der Zeichnung näher erläutert. Es zeigen:
Fig.1 eine Mehrfach- Blutbeutelanordnung für Spenderblut, bei der die einzelnen Beutel über Brechventile und Schläuche miteinander verbunden und in Schlauchbereichen photoaktive Substanzen eingeschlossen sind,
Fig.1a einen vergrößerten Ausschnitt A eines mit einer photoaktiven Substanz gefüllten Schlauchabschnittes,
Fig.2 eine Einfach-Blutbeutelanordnung, bestehend aus einem einzigen Beutel und einem Schlauch, mit im Schlauch eingeschlossenen photoaktiven Substanzen. Diese Blutbeutelanordnung ist als selbständige Einheit einzeln handhabbar und an eine ebenfalls als selbständige Einheit zu handhabende Mehrfach-Blutbeutelanordnung nach Fig.1 anschweißbar.

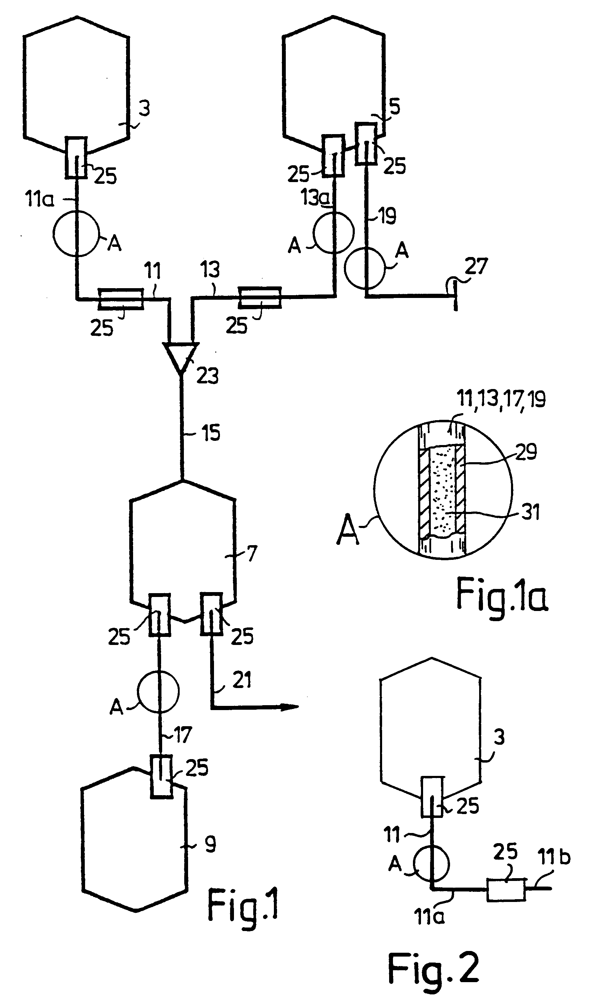

Fig. 1 zeigt eine Mehrfach-Blutbeutelanordnung mit zwei Satellitenbeuteln 3, 5, einem Abnahmebeutel 7 sowie einem Beutel 9 für eine Additivlösung. Der Satellitenbeutel 3 und der Entnahmebeutel 7 sowie der Satellitenbeutel 5 und der Entnahmebeutel 7 sind über Schläuche 11, 13 miteinander verbunden. An den Satellitenbeutel 5 ist ein als Pig-Tail bezeichnetes, am freien Ende 27 geschlossenes Schlauchende 19 angeordnet. An den Entnahmebeutel 7 schließt sich ein Entnahmeschlauch 21 an. Die von den Satellitenbeuteln 3 und 5 kommenden Schläuche 11 und 13 werden im Bereich eines Einwegventiles 23 zusammengeführt. Sie sind auf diese Weise über einen gemeinsamen Schlauch 15 mit dem Entnahmebebeutel 7 verbunden.

In dem Schlauch 11 gibt es ein Mittelstück 11a, welches im betriebsbereiten Zustand der Blutbeutelanordnung mittels Brechventilen 25 verschlossen ist. Ebenso gibt es im Schlauch 13 ein Mittelstück 13a, das im betriebsbereiten Zustand beidseitig mittels Brechventilen 25 verschlossen ist. Das an seinem freien Ende 27 abgeschlossene Schlauchende 19 ist an den Satellitenbeutel 5 mittels eines weiteren Brechventiles 25 angeschlossen.

Die nur schematisch dargestellten Brechventile sind von bekannter Bauart. Es handelt sich dabei um Kunststoffspritzteile mit einem Durchlaßkanal, dessen Durchgang nach der Herstellung zunächst mittels einer mitgespritzten Membran oder dergleichen blockiert ist. Die Brechventile sind sogenannte Einmalventile und derart ausgebildet, daß die Blockierung des Durchganges bei einem Knicken aufgehoben wird. Die Membran wird beim Knicken entfernt. Der Durchlaß ist dann offen, und durch den Durchlaßkanal kann ein Medienaustausch stattfinden.

Ein Schlauch 17 verbindet unmittelbar den Entnahmebeutel 7 mit dem Additivbeutel 9. Die Anschlüsse des Schlauches 17 an die Beutel 7 und 9 übernehmen Brechventile 25. Ein Entnahmeschlauch 21 ist über ein weiteres Brechventil 25 an den Entnahmebeutel angeschlossen.

In der Fig.1 sind mit Kreisen A bezeichnete Bereiche der Schläuche 11, 13, 19 und 17 hervorgehoben. Diese Bereiche A sind in Fig.1a stark vergrößert wiedergegeben. Damit wird ein Schnittbild der Schlauchbereiche dargestellt. Im Schnittbereich ist erkennbar, daß die Schlauchbereiche 11a,13a,17,19 zwischen den Schlauchwänden 29 eine photoaktive Substanz 31 enthalten. Die photoaktiven Substanzen 31 sind im betriebsbereiten Zustand zwischen den Brechventilen 25 in die Schlauchbereiche 11a,13a,17,19 eingeschlossen. Durch Brechen der Ventile 25 können die photoaktiven Substanzen in die jeweiligen Beutel 3, 5, 7, 9 einfließen.

Fig.2 zeigt eine Einfach-Blutbeutelanordnung mit einem Beutel 3, der mit einem Schlauch 11 versehen ist. Der Schlauch 11 mündet über ein Brechventil 25 in den Beutel 3. Weiterhin ist im Schlauch 11 in einem Abstand vom Brechventil 25 am Beutel 3 ein weiteres Brechventil 25 vorgesehen. Dadurch bildet sich ein abgeschlossener Schlauchabschnitt 11a aus. In dem abgeschlossenen Schlauchabschnitt 11a ist eine photoaktive Substanz 31 eingeschlossen. Dies ist anhand des Ausschnittes A angedeutet, der der Ausbildung nach Fig.1a entspricht. Das freie Ende 11b des Abgabeschlauches 11 ist zunächst frei.

Das freie Ende 11b des Abgabeschlauches 11 kann beispielsweise an einen Schlauch der Blutbeutelanordnung nach Fig.1 angekoppelt werden. Dazu bedient man sich beispielsweise einer Schweißvorrichtung, die das freie Ende 11b beispielsweise an den Schlauch 15 nach Fig.1 anschweißt und damit verbindet.

Die photaaktiven Substanzen werden im Rahmen der Erfindung als Flüssigkeiten 31 eingesetzt. Selbstverständlich ist es auch möglich, sie in fester Form (etwa als Pulver) einzusetzen.

Wenn sich das Blutplasma oder eine zelluläre Blutfraktion mit der photoaktiven Substanz vermischen soll, dann werden die Brechventile 25 durch Brechen geöffnet. Bei dieser Vorgehensweise bleibt die Beutelanordnung in sich geschlossen. Es entfällt somit jedes Risiko einer bakteriellen Infektion. Analog wären selbstverständlich auch noch andere Anordnungen mit mehr oder weniger Beuteln in der gleichen Weise mit photoaktiven Substanzen ausrüstbar.

Als photoaktive Substanzen kommen beispielsweise Phenothiazinfarbstoffe in Betracht. Unter diesen sind wiederum das Methylenblau und Toluidinblau am geeignetsten.

## Patentansprüche

1. Blutbeutelanordnung für Spenderblut und/ oder Blutprodukte mit einem Beutel (3), zumindest einem an diesen angeschlossenen Schlauch (11) und einer Einrichtung zur Inaktivierung von Viren, wobei die Anordnung aus zumindest teilweise lichtdurchlässigem Kunststoffmaterial besteht, dadurch gekennzeichnet, daß in einem abgeschlossenen Bereich (A) des Schlauches (11) der benutzungsbereiten Anordnung, die als selbstständige Einheit einzeln handhabbar ist, photoaktive Substanzen (31) einschließbar sind.

2. Blutbeutelanordnung für Spenderblut und/oder Blutprodukte mit mehreren Beuteln (3,5,7,9) und der an angeschlossenen Schläuchen (11,13,15,17), die aus zumindest teilweise lichtdurchlässigem Kunststoffmaterial bestehen, wobei die Schläuche (11,13,15,17) die Beutel (3,5,7,9) miteinander verbinden und/oder an anderweitige Schläuche (19,21) angeschlossen sind, dadurch gekennzeichnet, daß in abgeschlossenen Bereichen (A) der Schläuche (11,13,15) der benutzungsbereiten Anordnung, die als selbständige Einheit einzeln handhabbar ist, photoaktive Substanzen (31) einschließbar sind.

3. Blutbeutelanordnung für Spenderblut und/oder Blutprodukte mit zumindest einem Beutel, zumindest einem angeschlossenen Schlauch, über den die Beutel weiterverbindbar sind und einer Einrichtung zur Inaktivierung von Viren, wobei die Anordnung zumindest teilweise aus lichtdurchlässigem Kunststoffmaterial besteht, dadurch gekennzeichnet, daß eine selbstständig und einzeln zu handhabende Blutbeutelanordnung, bestehend aus einem Beutel (3) und einem in abgeschlossenen Bereichen (A) photoaktive Substanzen (31) einschließenden Schlauch (11), mit einer anderen selbständig und einzeln zu handhabenden Blutbeutelanordnung, die aus einem Beutel (3) und einem Schlauch (11) oder aus mehreren Beuteln (3,5,7,9) und die Beutel verbindenden Schläuchen (11,13,15,17) besteht, kombinierbar ist durch ein Miteinanderverbinden dieser Blutbeutelanordnungen.

4. Blutbeutelanordnung nach Anspruch 2 oder 3, dadurch gekennzeichnet, daß bei der Mehrbeutelanordnung Entnahmebeutel (7) und weitere Beutel (3,5,9) über Substanzen (31) einschließenden Schlauche (11,13,15,17) miteinander verbunden sind.

5. Blutbeutelanordnung nach einem der Ansprüche 1 bis 3, dadurch gekennzeichnet, daß die Schläuche (11) der einen Blutbeutelanordnung an Schlauchleitungen (11,13,15,17,21) der anderen Blutbeutelanordnung anschweißbar sind.

6. Blutbeutelanordnung nach einem der Ansprüche 1 bis 5, dadurch gekennzeichnet, daß die photoaktiven Substanzen (31) im flüssigen bzw. gelösten Zustand vorgesehen sind.

7. Blutbeutelanordnung nach einem der Ansprüche 1 bis 4, dadurch gekennzeichnet, daß als photoaktive Substanzen (31) Phenothiazinfarbstoffe vorgesehen sind.

8. Blutbeutelanordnung nach Anspruch 7, dadurch gekennzeichnet, daß als Farbstoffe Methylenblau oder Toluidinblau vorgesehen sind.

## Claims

1. A blood bag arrangement for donor blood and/or blood products having a bag (3), at least one tube (11) attached to it and means for the inactivation of viruses, the arrangement consisting of at least partly transparent plastic material, characterized in that photoactive substances (31) can be enclosed in a closed area (A) of the tube (11) of the arrangement ready for use which can be handled separately as independent unit.

2. A blood bag arrangement for donor blood and/or blood products having several bags (3, 5, 7, 9) and tubes (11, 13, 15, 17) attached to them which consist of at least partly transparent plastic material, the tubes (11, 13, 15, 17) connecting the bags (3, 5, 7, 9) together and/or being attached to other tubes (19, 21), characterized in that photoactive substances (31) can be enclosed in closed areas (A) of the tubes (11, 13, 15) of the arrangement ready for use which can be handled separately as independent unit.

3. A blood bag arrangement for donor blood and/or blood products having at least one bag, at least one tube attached to it by which the bags can be further connected, and means for the inactivation of viruses, the arrangement consisting of at least partly transparent plastic material, characterized in that a blood bag arrangement which can be handled independently and separately, consisting of one bag (3) and one tube (11) which includes photoactive substances (31) in closed areas (A), is able to be combined with another blood bag arrangement by connecting together these blood bag arrangements, the other blood bag arrangement being able to be handled independently and separately, consisting of one bag (3) and one tube (11) or of several bags (3, 5, 7, 9) and tubes (11, 13, 15, 17) connecting the bags.

4. The blood bag arrangement according to claim 2 or 3, characterized in that in a multiple bag arrangement discharge bag (7) and further bags (3, 5, 9) are connected together by tubes (11, 13, 15, 17) enclosing substances (31).

5. The blood bag arrangement according to one of claims 1 to 3, characterized in that the tubes (11) of one blood bag arrangement can be welded on to tubings (11, 13, 15, 17, 21) of the other blood bag arrangement.

6. The blood bag arrangement according to one of claims 1 to 5, characterized in that the photoactive substances (31) are provided in the liquid or dissolved state, respectively.

7. The blood bag arrangement according to one of claims 1 to 4, characterized in that phenothiazine dyes are provided as photoactive substances (31).

8. The blood bag arrangement according to claim 7, characterized in that methylene blue or toluidine blue are provided as dyes.

## Revendications

1. Système à poche à sang pour sang de donneur et/ou produits sanguins, comprenant une poche (3), au moins un flexible (11) raccordé à celle-ci et un dispositif d'inactivation de virus, le système étant en matière plastique au moins partiellement transparente, caractérisé en ce que des substances photo-actives (31) peuvent être enfermées dans une zone fermée (A) du flexible (11) du système prêt à l'emploi et se manipulant individuellement sous forme d'un module autonome.

2. Système à poches à sang pour sang de donneur et/ou produits sanguins, comprenant plusieurs poches (3, 5, 7, 9) et des flexibles (11, 13, 15, 17) raccordés à celles-ci, qui sont en matière plastique au moins partiellement transparente, les flexibles (11, 13, 15, 17) raccordant les poches (3, 5, 7, 9) les unes aux autres et/ou à d'autres flexibles (19, 21), caractérisé en ce que des substances photo-actives (31) peuvent être enfermées dans des zones fermées (A) des flexibles (11, 13, 15) du système prêt à l'emploi et se manipulant individuellement sous forme d'un module autonome.

3. Système à poches à sang pour sang de donneur et/ou produits sanguins, comprenant au moins une poche, au moins un flexible raccordé par lequel les poches peuvent être reliées par ailleurs, ainsi qu'un dispositif d'inactivation de virus, le système étant en matière plastique au moins partiellement transparente, caractérisé en ce qu'un système autonome à poche à sang se manipulant individuellement et se composant d'une poche (3) et d'un flexible (11) renfermant dans des zones fermées (A) des substances photoactives (31) peut être combiné avec un autre système autonome à poche à sang se manipulant individuellement, se composant d'une poche (3) et d'un flexible (11) ou de plusieurs poches (3, 5, 7, 9) et de flexibles (11, 13, 15, 17) reliant les poches, par raccordement de ces systèmes à poche à sang l'un à l'autre.

4. Système à poches à sang selon la revendication 2 ou 3, caractérisé en ce que, dans le système à plusieurs poches, une poche de prélèvement (7) et d'autres poches (3, 5, 9) sont raccordées les unes aux autres par des flexibles (11, 13, 15, 17) renfermant des substances (31).

5. Système à poche à sang selon l'une des revendications 1 à 3, caractérisé en ce que les flexibles (11) de l'un des systèmes à poche à sang sont raccordés par soudure à des conduits flexibles (11, 13, 15, 17, 21) de l'autre système à poches à sang.

6. Système à poche à sang selon l'une des revendications 1 à 5, caractérisé en ce que les substances photo-actives (31) qui sont prévues sont à l'état liquide ou en solution.

7. Système à poche à sang selon l'une des revendications 1 à 4, caractérisé en ce que les substances photo-actives (31) qui sont prévues sont des colorants à base de phénothiazine.

8. Système à poche à sang selon la revendication 7, caractérisé en ce que les colorants prévus sont du bleu de méthylène ou du bleu de toluidine.
